# EUROPEAN PATENT APPLICATION

(11) **EP 2 963 029 A1**
(43) Date of publication of application: **06.01.2016**
(21) Application number: 14175553.8
(22) Date of filing: 03.07.2014
(51) Int. Cl.: C07D 309/30, A61K 31/366, A61P 29/00, A61P 31/00

(54) **Cyclic derivatives of epoxyisoprostanoids as therapeutic agents**

(71) Applicant: ETH Zurich, 8092 Zurich (CH)
(72) Inventor: Carreira, Erick, 8126 Zumikon (CH); Egger, Julian, 86179 Augsburg (DE); Kopf, Manfred, 8006 Zürich (CH); Bretscher, Peter, 8046 Zürich (CH); Freigang, Stefan, 8057 Zürich (CH)

(57) **Abstract**

The invention relates to cyclic derivatives of epoxyprostanoids having a lactone ring, in particular to compound 9 and related compounds, and their use as therapeutic agents in the treatment of bacterial infections and inflammation.

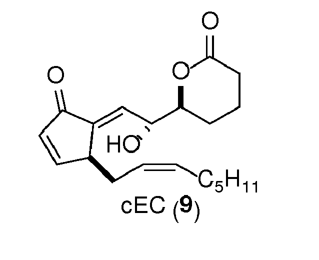

## Description

### Field of the invention

The invention relates to cyclic derivatives of epoxyprostanoids having a lactone or other heterocyclic ring and their use as therapeutic agents in the treatment of bacterial infections and inflammation.

### Background of the invention

Oxidized phospholipids (OxPLs) are a class of biomolecules that have gained increasing attention as important and highly potent regulators of various physiological functions within higher organisms (Bochkov, V.N. et al., J. Antioxid. Redox Signaling 2010, 12, 1009). Among the different classes of OxPLs, the epoxyisoprostanes, such as EC (**8**) and EI (**19**) are particularly interesting, as their phosphatidylcholine bound congeners have been shown to play a pivotal role in the early development of atherosclerosis and other inflammatory conditions (Subbanagounder, G. et al., J. Biol. Chem. 2002, 277, 7271). A rapid and efficient synthesis of the synthetically challenging epoxyisoprostanes **8** and **19** was described recently, and unprecedented *anti*-inflammatory effects discovered, namely the reduced secretion of *pro*-inflammatory cytokines IL-6 and IL-12 in bone-marrow derived dendritic cells (Egger, J.; Bretscher, P.; Freigang, S.; Kopf, M.; Carreira, E. M., Angew. Chem. Int. Ed. 2013, 52, 5382). Moreover, the free acids 5,6-epoxyisoprostane A₂ (EC (**8**)) and 5,6-epoxyisoprostane E₂ (EI (**19**)) were shown to be more potent than their phospholipid bound congeners and that EC (**8**) is more potent than EI (**19**).

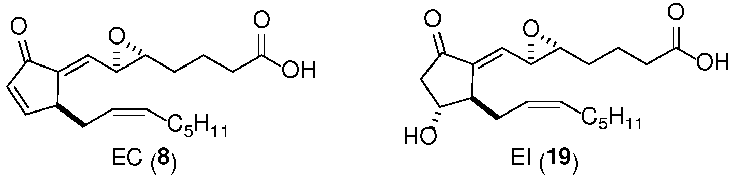

### Summary of the invention

The invention relates to cyclic derivatives of epoxyprostanoids of formula (I) and (II) 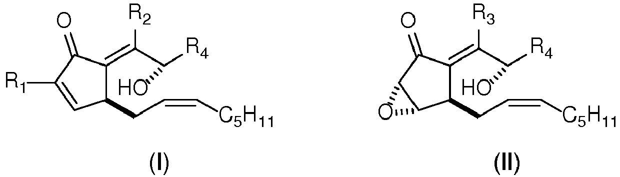 wherein
R₁, R₂ and R₃ are, independently of each other, hydrogen, fluoro, chloro, nitro or trifluoromethyl;
R₄ is of partial formula 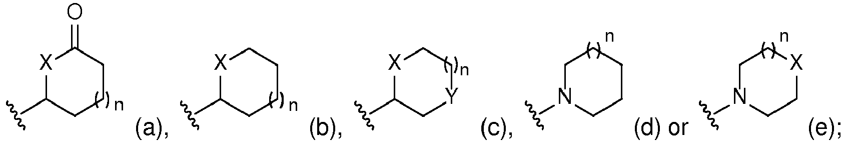
X and Y, are independently of each other, O or NR₅;
R₅ is hydrogen or C₁-C₄-alkyl; and
n is 0 or 1;
pharmaceutical compositions comprising these compounds, the use of these compounds for the treatment of inflammatory diseases, a method of synthesis of such compounds, and a method of treating infectious and inflammatory diseases comprising administering these compounds to a patient in need thereof.

### Brief Description of the Figures

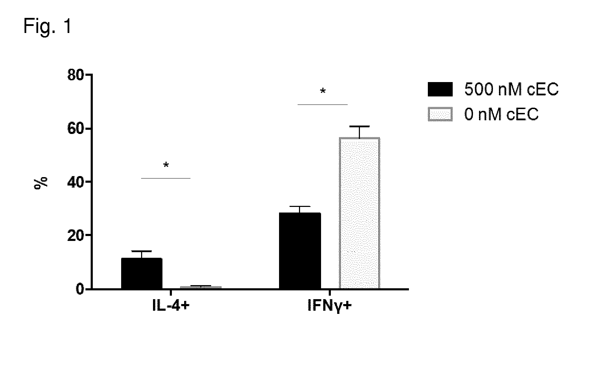

Figure 1: Treatment with cyclo-EC (**9**) licenses dendritic cells to polarize niave CD4 T cells towards the Th2 subset.
   Naïve transgenic splenic CD4 T cells were co-cultured together with splenic dendritic cells that had been pre-treated either with cyclo-EC (**9**) (cEC) or the respective solvent control. CD4 T cells were then stimulated with the cognate antigen for 4 days. Treatment with cEC interferes with high dose antigen induced polarization towards the Th1 subset and licenses the dendritic cells to promote polarization of the Th2 subset instead. Shown is the percentage of IL-4 producing Th2 and IFNγ producing Th1 cells after treatment with cEC or solvent DMSO. Differences between cEC and DMSO treatments are significant as indicated by (*) using unpaired, two-tailed t tests. IL-4 producers (p = 0.039) and IFNγ producers (p = 0.0161).

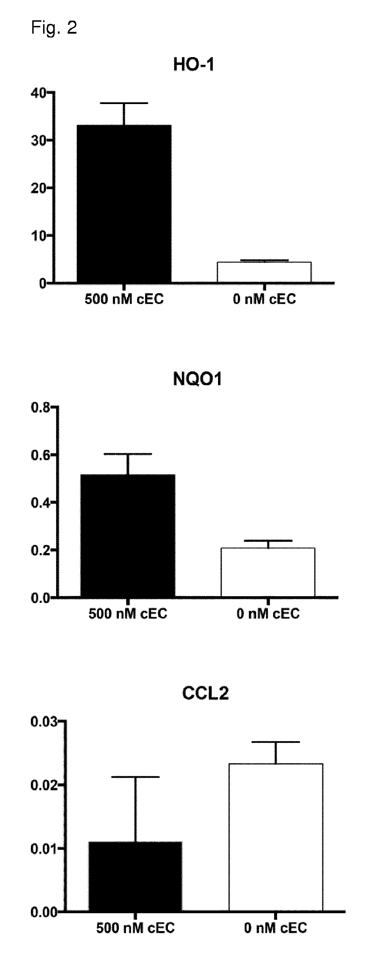

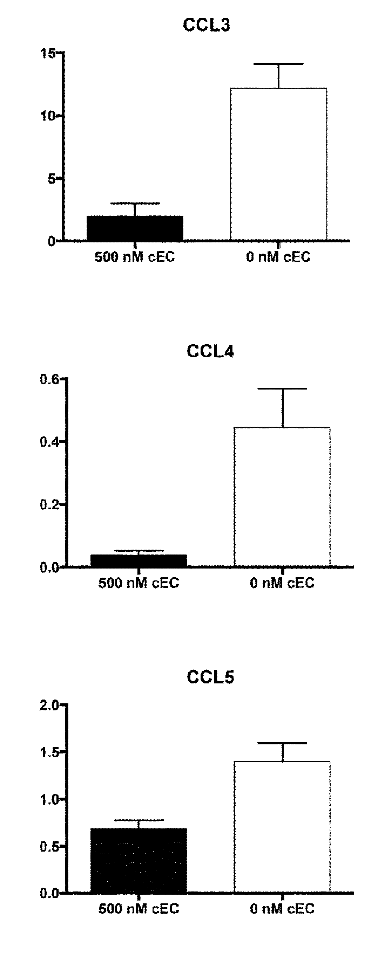

Figure 2: cyclo-EC (**9**) induces expression of Nrf2 target genes and inhibits pro-inflammatory chemokine expression in myeloid cells in vitro.
   Wild type bone marrow-derived dendritic cells were pretreated with cyclo-EC (**9**) (cEC) or solvent control for 75 minutes before stimulation with the TLR7 (toll-like receptor 7) ligand R837 (Syed, T. A., Exp.Opin.Pharmacother. 2001, 2, 877) for an additional 3 h. Cells were then lysed, mRNA was purified, reverse transcribed and expression of Nrf2 (nuclear factor like 2) target genes as well as the indicated chemokine genes was determined by qPCR. Expression levels were normalized to G6PDH (glucose 6-phosphate dehydrogenase) expression.
   Nrf2 target genes: HO-1 (heme oxygenase-1) and NQO1 (NAD(P)H quinone oxidoreductase 1)
   Pro-inflammatory chemokine genes: CCL1, 2, 3 and 4 (chemokine (CC motif) ligands)
Figure 3. Testing bioactivity of epoxycyclopentenone analogs: Crucial role of an endocyclic electrophile in IL-6 and IL-12 inhibition.
   Bone marrow-derived dendritic cells (BMDCs) were treated with (**8**), (**10**), (**11**), (**12**), (**13**) and (**14**) for 60 minutes and subsequently stimulated with TLR7 agonist R837 for 18 h. Cytokines in the supernatant were quantified with ELISA.
Figure 4. Testing bioactivity of a second set of epoxycyclopentenone analogs: Identification of cyclo-EC (**9**) as a potent IL-6 / IL-12 inhibitor.
   Bone marrow-derived dendritic cells (BMDCs) were treated with indicated lipids for 60 minutes and subsequently stimulated with TLR7 agonist R837 for 18 h. Cytokines in the supernatant were quantified with ELISA.
Figure 5. Direct comparison of the impact of EC (**8**) and cyclo-EC (**9**) on IL-6 and IL-12 secretion.
   Bone marrow-derived dendritic cells (BMDCs) were treated with indicated lipids for 60 minutes and subsequently stimulated with TLR7 agonist R837 for 18 h. Cytokines in the supernatant were quantified with ELISA. cEC attenuates secretion of pro-inflammatory cytokines at lower concentrations than EC and therefore proves to be more potent than EC.

### Detailed description of the invention

The invention relates to cyclic derivatives of epoxyprostanoids of **formula** (I) and (II) 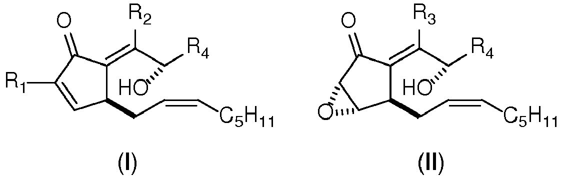 wherein
R₁, R₂ and R₃ are, independently of each other, hydrogen, fluoro, chloro, nitro or trifluoromethyl;
R₄ is of partial formula 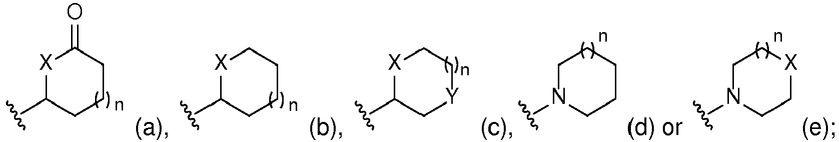
X and Y, are independently of each other, O or NR₅;
R₅ is hydrogen or C₁-C₄-alkyl; and
n is 0 or 1.

Preferably, R₄ is of partial formula (a), in particular wherein X is O and n is 0 or 1, preferably 1.

More preferably, the invention relates to compounds of formula (**III**) and (**IV**) 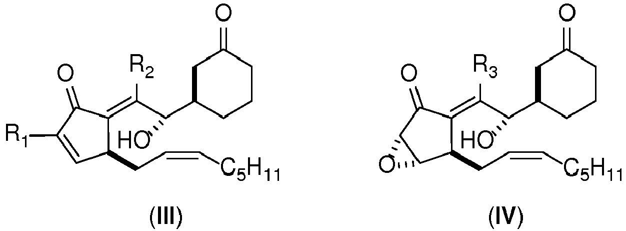 wherein R₁, R₂ and R₃ are, independently of each other, hydrogen, fluoro, chloro, nitro or trifluoromethyl. Preferred are compounds of formula (**III**) wherein R₁ is hydrogen, fluoro, chloro, nitro or trifluoromethyl, in particular hydrogen, fluoro or chloro, and R₂ is hydrogen. Also preferred are compounds of formula (**IV**), wherein R₃ is hydrogen, chloro or fluoro, in particular hydrogen. Most preferred is the compound of formula (**I**), wherein R₁ and R₂ are hydrogen. This compound is called cyclo-EC (cEC) and has formula **9**.

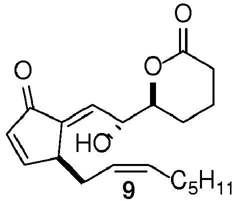

The invention is based on the observation of the bioactivity of oxidized 1-palmitoyl-2-arachidonoyl phosphatidylcholine (oxPAPC). *In vitro* oxidation of the abundant PAPC was followed by assessment of the bioactivity of the array of lipid modification products generated during this process by various bio-assays. Subsequently, mass spectrometry of these oxidized lipids revealed candidate products that appeared to be the mediators of the biological effects observed. Testing these promising candidate lipids in an isolated fashion confirmed strong activities of the oxidized PAPC products EI (**19**) and EC (**8**).

Further testing and generation of various derivatives of EC (**8**) lead to the discovery of an even more potent lipid containing a 6-membered lactone ring, cyclo-EC (cEC) of formula **9**.

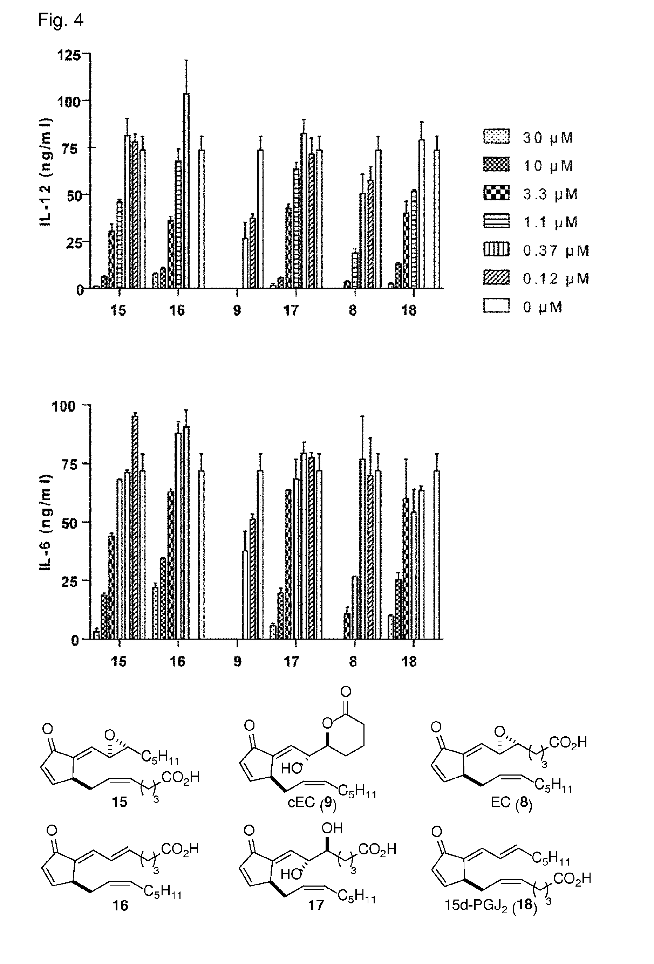

Earlier experiments using the oxidized modification product EC (**8**) revealed that it acts as a potent inhibitor of toll-like receptor 7 (TLR) mediated secretion of pro-inflammatory cytokines in myeloid cells. Moreover, EC (**8**) was able to license splenic dendritic cells to polarize naïve CD4 T cells towards the Th2 subset. Furthermore, using mice deficient in various transcription factors indicated that this suppressive effect in IL-6 and IL-12 secretion is dependent on the presence of Nrf2. Quantitative PCR revealed that EC was able to drive expression of downstream targets of Nrf2. Repeating these experiments with the more potent cEC (**9**) indicates that it shows the same bioactivity in promoting Th2 polarization (Fig. 1), the up-regulation of Nrf2 downstream target genes (Fig. 2), as well as the suppression of pro-inflammatory cytokines IL-6 and IL-12 (Fig. 4-5).

The synthetic route described below substantially differs from previously reported routes to epoxyisoprostanes. In general, the existing synthetic approaches commence from cyclopentadiene and require desymmetrization of this 5-membered ring. Subsequent conversion into a cyclopentenone allowed for installation of nucleophilic ω-chain fragments, which then had to be elongated to yield the full chain on the cyclopentanone core.

The synthesis of the present invention (Scheme 1) starts from commercially available acyclic aldehyde **1** which contains the full pattern of the ω-side chain present in the final product. C₂-elongation of the aldehyde and concomitant installation of the C11 stereocenter furnished β-lactone **2** which was subsequently opened with methyl acetate enolate to afford **3**. Diazotransfer and protection of the secondary alcohol furnished **4** which underwent Rh₂(S-PTAD)₄-catalyzed carbene insertion into the homoallylic C-H bond to give cyclopentanone **5** as the sole product in a 9:1 diastereomeric mixture. This insertion was unprecedented when the full ω-side chain is in place. Decarboxylation and elimination of the TES-silanol led to cyclopentenone **6**, on which the α-side chain was installed by means of an aldol reaction/elimination with epoxy aldehyde **7**. The existing procedure according to Kobayashi (Angew. Chem. Int. Ed, 2005, 44, 3481) for this step, suffered from the high and unpredictable instability of the intermediate mesylate. In the present procedure the isolation of the mesylate is not necessary anymore. Hence, the yields of elimination product is considerably improved. Finally, enzymatic saponification under neutral conditions with *Novozyme®* gave EC (**8**) in 10 steps and 7.8% overall yield. Treatment of the free acid EC (**8**) with silica gel in CHCl₃ afforded cyclo-EC (**9**) in 65% yield. 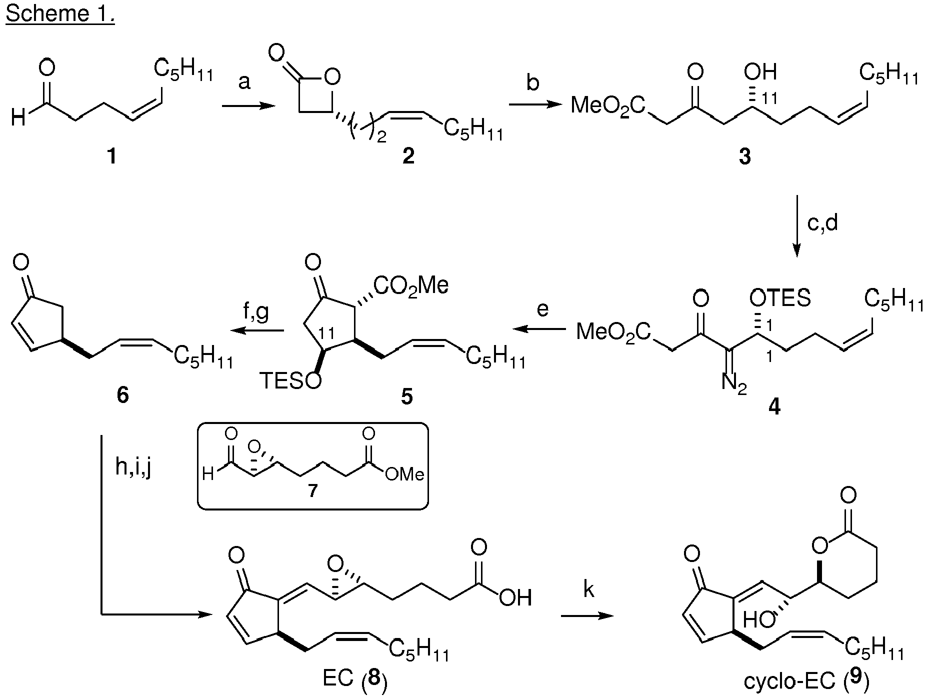 Reagents and conditions: a) LiClO₄ (3 equiv.), Me₃Si-quinidine (12 mol%), *i*-Pr₂NEt (2.5 equiv.), AcCl (2.5 equiv., over 4 h *via* syringe pump), Et₂O/CH₂Cl₂, -78°C, 62%, 92% *ee*; b) *i*-Pr₂NLi (3.8 equiv), methyl acetate (3.8 equiv.), THF, -78°C, 77%; c) *p*-ABSA (1.3 equiv.), Et₃N (2 equiv.), MeCN, 0°C to RT, 97%; d) Et₃SiCl (1.5 equiv.), imidazole (2 equiv.), DMF, 0°C to RT, 98%; e) Rh₂(S-PTAD)₄ (1 mol%), CH₂Cl₂, reflux, d.r. = 9:1, 71%; f) NaCl (30 equiv.), Me₂SO, 140°C, 65%; g) DBU (10 equiv.), CH₂Cl₂, 0°C, 93%; h) LiN(SiMe₃)₂ (1.2 equiv.), then **7** (2 equiv.), THF, -78°C; i) MeSO₂Cl (3 equiv.), Et₃N (6 equiv), CH₂Cl₂, -78°C then Al₂O₃, CH₂Cl₂, RT, 64% over two steps; j) Novozyme®, buffer pH 7/THF, 70%; k) SiO₂, CHCl₃, room temperature.
*p*-ABSA = *para*-acetamidobenzenesulfonyl azide, S-PTAD = S-(1-adamantyl)-(N-phthalimido)-acetato, DBU = 1,8-diazabicyclo[5.4.0]undec-7-ene, Novozyme® = lipase on acrylic resin from *Candida antarctica*.

The present invention also relates to a method of synthesis of compounds of formula (**III**) and (IV) wherein a compound of formula (**V**) and (**VI**), respectively, 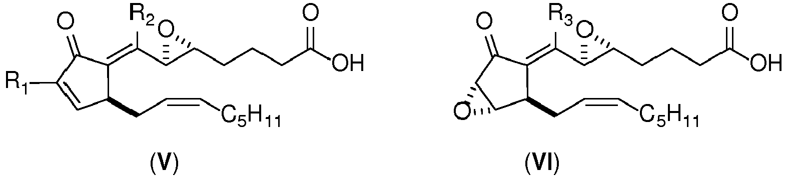 wherein R₁, R₂ and R₃ have the meaning indicated for formula (**III**) and (**IV**), is treated with SiO₂, in particular with SiO₂ as a slurry in CHCl₃.

Compounds of formula (**I**) or (**II**), wherein
R₄ is of partial formula 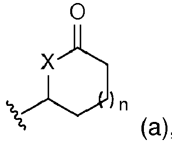 n is 0 and X is O are prepared from the corresponding compounds of formula (**V**) and (**VI**) wherein the side chain carrying the carboxy function is shortened by one carbon atom. For the preparation of compounds of formula (**I**) or (**II**), wherein R₄ is of partial formula (a), n is 0 or 1 and X is NR₅, the corresponding open chain carboxamide (-CONR₅) is cyclized by Bronsted or Lewis acid treatment in a variety of solvents.

Compounds of formula (**I**) or (**II**), wherein
R₄ is of partial formula 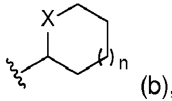 n is 0 or 1 and X is O or NR₅, are prepared from the intermediate of formula **6** in an aldol addition with an aldehyde of the structure 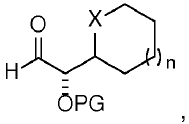 wherein PG means a standard protecting group for hydroxy, that itself can be prepared from the corresponding vinyl heterocycle 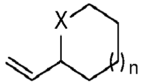 by asymmetric dihydroxylation followed by selective protection of the secondary and oxidation of the primary alcohol.

Compounds of formula (**I**) or (**II**), wherein
R₄ is of partial formula 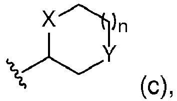 n is 0 or 1, X is O or NR₅ and Y is O or NR₅, are prepared from the intermediate of formula **6** in an aldol addition with an aldehyde of the structure 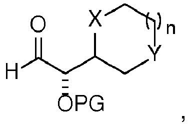 wherein PG means a standard protecting group for hydroxy, that itself can be prepared from the corresponding vinyl heterocycle 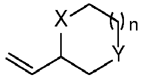 by asymmetric dihydroxylation followed by selective protection of the secondary and oxidation of the primary alcohol.

Compounds of formula (**I**) or (**II**), wherein
R₄ is of partial formula 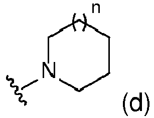 and n is 0 or 1, are prepared from the intermediate of formula **6** in an aldol addition with an aldehyde of the structure 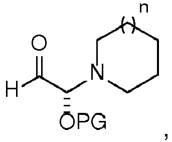 wherein PG means a standard protecting group for hydroxy, that itself can be prepared from the corresponding vinyl heterocycle 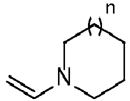 by asymmetric dihydroxylation followed by selective protection of the secondary and oxidation of the primary alcohol.

Compounds of formula (**I**) or (**II**), wherein
R₄ is of partial formula 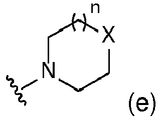 and n is 0 or 1, X is O or NR₅, are prepared from the intermediate of formula 6 in an aldol addition with an aldehyde of the structure 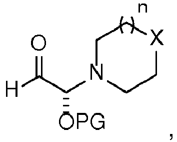 wherein PG means a standard protecting group for hydroxy, that itself can be prepared from the corresponding vinyl heterocycle 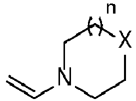 by asymmetric dihydroxylation followed by selective protection of the secondary and oxidation of the primary alcohol.

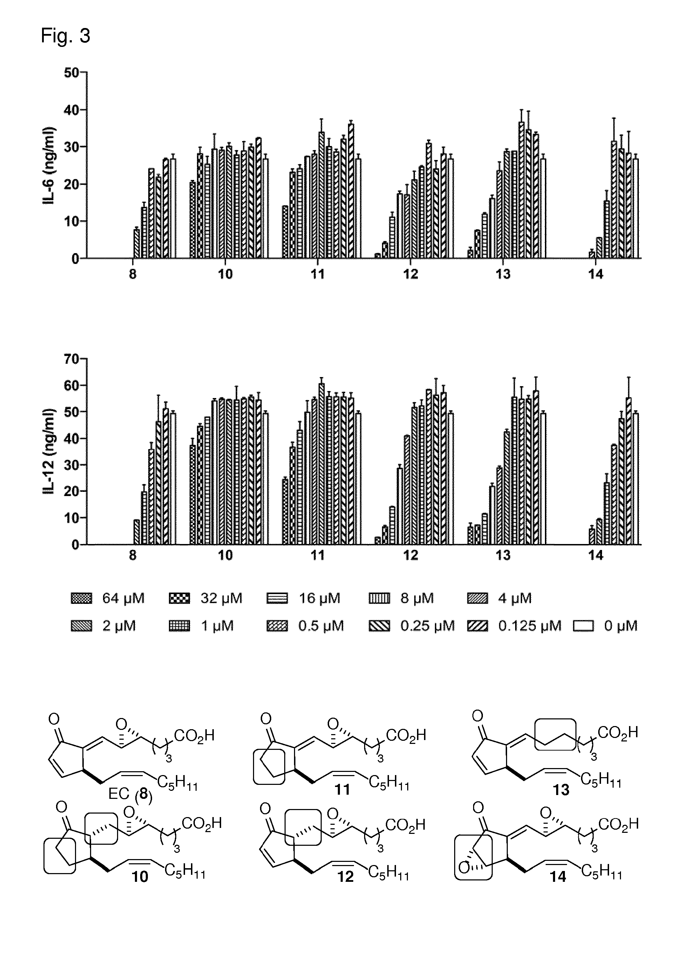

Structure-activity-relationship studies helped to define the active sites of EC required for its bioactivity. This culminated in the discovery of the highly potent EC-derived lactone cyclo-EC (**9**). A first series of analogs investigated the role of the candidate compounds as electrophiles and revealed that the endocyclic enone is crucial for the observed biological activity and that the exocyclic enone and the epoxide have reinforcing character (Figures 3 and 4).

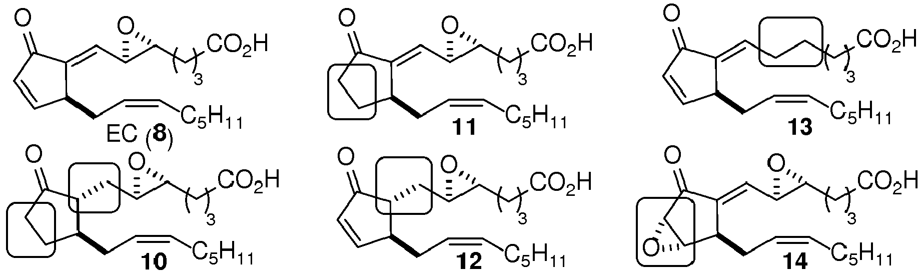

This result stands in contrast to the observations made by Jung (*Org. Lett.,* **2008**, *10*, 4207). The finding, however, does not explain the difference in potency between EC (**8**) and 15-deoxy-Δ^{12,14}-prostaglandin J2 (**18**), as both compounds contain an endo- and exocyclic enone. The presence of an activated allylic epoxide and a carboxylic acid in the same side chain of **8** reveals an interaction between these two moieties and its impact on the bioactivity. Such an interaction, with concomitant opening of the epoxide by the carboxylic acid, results in a thermodynamically favored δ-lactone. A second set of EC-analogs was designed in order to confirm this observation.

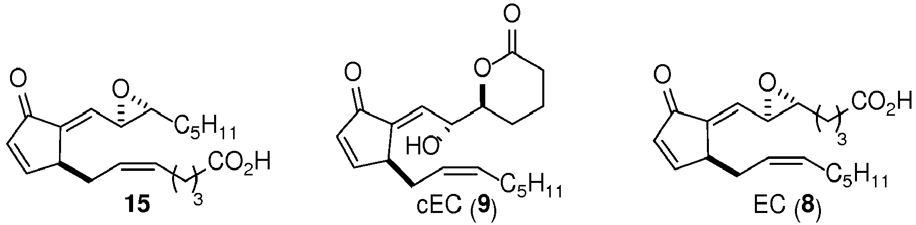

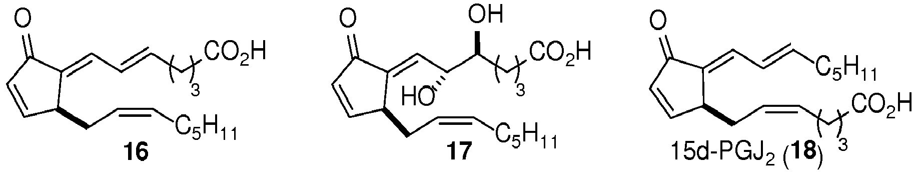

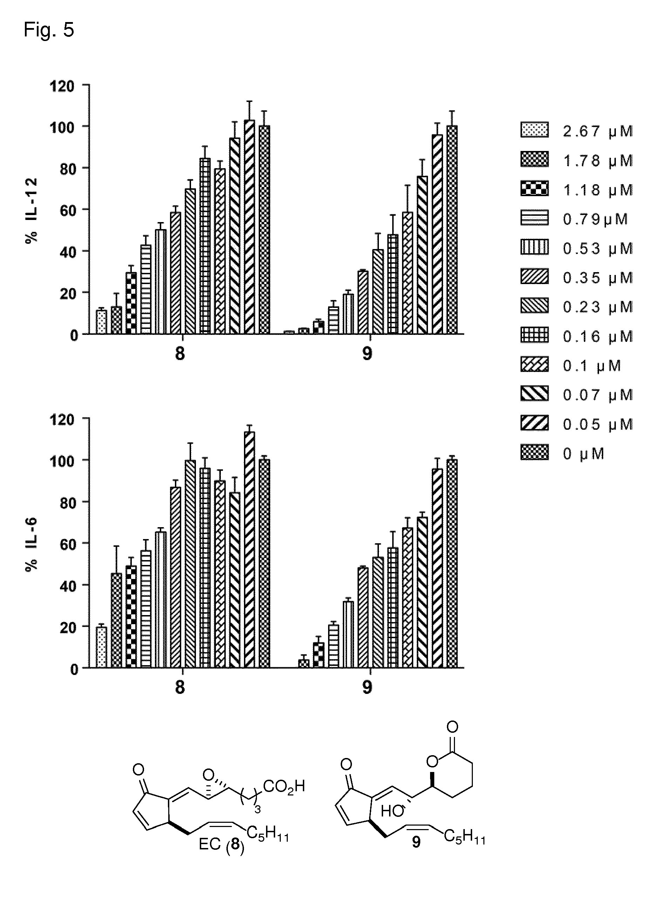

The lactone ring containing cyclo-EC (**9**) was the most potent inhibitor of IL-6 and IL-12 secretion (Figure 5), and cyclo-EC (**9**) is even more potent than parent EC (8). The high activity of bis-epoxy compound **14** indicates that further derivatives that combine the epoxyde and the lactone moiety might be of even higher potency.

Altogether, these results directly demonstrate that cyclo-EC (**9**) inhibits several aspects linked to detrimental inflammatory responses *in vivo,* and thus represents a promising candidate compound with great potential for anti-inflammatory immunotherapy.

Furthermore the invention relates to a pharmaceutical composition comprising the compound of formula (**I**) or (**II**) as defined hereinbefore.

Pharmaceutical compositions for parenteral administration, such as subcutaneous, intravenous, intrahepatic or intramuscular administration, to warm-blooded animals, especially humans, are considered. The compositions comprise the active ingredient(s) alone or, preferably, together with a pharmaceutically acceptable carrier. The dosage of the active ingredient(s) depends upon the age, weight, and individual condition of the patient, the individual pharmacokinetic data, and the mode of administration.

For parenteral administration preference is given to the use of suspensions or dispersions of the compounds of the invention, especially isotonic aqueous dispersions or suspensions which, for example, can be made up shortly before use. The pharmaceutical compositions may be sterilized and/or may comprise excipients, for example preservatives, stabilizers, wetting agents and/or emulsifiers, solubilizers, viscosity-increasing agents, salts for regulating osmotic pressure and/or buffers and are prepared in a manner known *per se*, for example by means of conventional dissolving and lyophilizing processes.

Suitable carriers for enteral administration, such as nasal, buccal, rectal or oral administration, are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient(s).

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

The mentioned pharmaceutical compositions according to the invention may contain separate tablets, granules or other forms of orally acceptable formulation of the active ingredients, or may contain a mixture of active ingredients in one suitable pharmaceutical dosage form, as described above. In particular the separate orally acceptable formulations or the mixture in one suitable pharmaceutical dosage form may be slow release and controlled release pharmaceutical compositions.

The pharmaceutical compositions comprise from approximately 1% to approximately 95% active ingredient or mixture of active ingredients, single-dose administration forms comprising in the preferred embodiment from approximately 20% to approximately 90% active ingredient(s) and forms that are not of single-dose type comprising in the preferred embodiment from approximately 5% to approximately 20% active ingredient(s).

The invention also relates to the mentioned pharmaceutical compositions as medicaments in the treatment of bacterial infections and inflammation.

The present invention relates furthermore to a method of treatment of bacterial infection and inflammation, which comprises administering a composition according to the invention in a quantity effective against said disease, to a warm-blooded animal requiring such treatment. Particular inflammatory disease considered are psoriasis, atopic dermatitis, inflammatory bowel disease, arthritis (rheumatoid arthritis, osteoarthritis), sepsis, asthma, sarcoidosis, acute respiratory distress syndrome, diabetes mellitus, multiple sclerosis, cancer, atherosclerosis, vasculitis, glomerulonephritis, and steatosis.

The pharmaceutical compositions can be administered prophylactically or therapeutically, preferably in an amount effective against the said diseases, to a warm-blooded animal, for example a human, requiring such treatment. In the case of an individual having a bodyweight of about 70 kg the daily dose administered is from approximately 0.01 g to approximately 5 g, preferably from approximately 0.25 g to approximately 1.5 g, of the active ingredients in a composition of the present invention.

The following Examples serve to illustrate the invention without limiting the invention in its scope.

### Examples

### Synthesis of cyclo-EC (9)

### Example 1: (R,Z)-4-(Non-3-en-1-yl)oxetan-2-one (2)

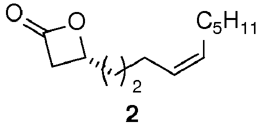

To a 1 L flask equipped with a big stirring bar and dried lithium perchlorate (22.06 g, 207 mmol, 3.00 equiv.) was added Et₂O (185 mL), followed by a solution of TMS-quinidine (Calter, M. A., J. Org. Chem. 1996, 61, 8006-8007; 3.38 g, 8.52 mmol, 0.12 equiv.) in CH₂Cl₂ (300 mL). The mixture was stirred at room temperature until a homogenous solution had formed and then cooled to -78°C before *i*-Pr₂NEt (30.1 mL, 173 mmol, 2.50 equiv.) was added, followed by a solution of decenal **1** (10.66 g, 69.1 mmol, 1.00 equiv.) in CH₂Cl₂ (160 mL). A solution of acetyl chloride (9.83 mL, 138 mmol, 2.50 equiv.) in CH₂Cl₂ (2 x 20 mL) was then added over 4 h *via* a syringe pump and the mixture kept stirring at -78°C for 8 h. After full consumption of the starting material the now yellow, turbid reaction mixture was quenched at-78°C with Et₂O (100 mL) and then allowed to reach room temperature, before it was filtered through a pad of silica, that was afterwards carefully washed with Et₂O (3 x 150 mL). The filtrate was concentrated *in vacuo* and the crude material purified by flash column chromatography (SiO₂, pentane/Et₂O, 4:1) to give β-lactone **2** (8.41 g, 42.8 mmol, 62%, 92% *ee*) as a colorless oil.

**TLC:** *R_{f}* = 0.41 (hexane/Et₂O, 3:1, vanilline). **¹H NMR** (400 MHz, CDCl₃): δ = 5.55 - 5.22 (m, 2H), 4.60 - 4.43 (m, 1 H), 3.51 (dd, *J* = 16.3, 5.8 Hz, 1 H), 3.08 (dd, *J* = 16.3, 4.3 Hz, 1 H), 2.19 (q, *J* = 7.3 Hz, 2H), 2.11- 1.89 (m, 3H), 1.86 - 1.73 (m, 1 H), 1.44 - 1.22 (m, 6H), 0.89 (t, *J* = 6.9 Hz, 3H). **¹³C NMR** (100 MHz, CDCl₃): δ = 168.33, 132.17, 127.10, 70.89, 43.06, 34.84, 31.63, 29.41, 27.31, 22.91, 22.69, 14.20. **IR** (thin film): 2927, 2854, 1827, 1453, 1407, 1375, 1278, 1199, 1125, 862, 826 cm⁻¹. **HRMS** (EI): exact mass calculated for C₁₂H₂₀O₂ [M⁺]: 196.1463; found: 196.1460. **[α]_{D}^{30°C} =** + 26.0 (c = 0.98, CHCl₃).

The enantiomers were separated by SFC: (100% CO₂, 2.5 mL/min, 25°C, chiracel OJ-H), retention times 5.5 and 6.1 min. The desired enantiomer was obtained in 92% enantiomeric excess.

### Example 2: (R,Z)-Methyl 5-hydroxy-3-oxotetradec-8-enoate (3)

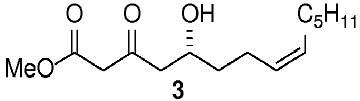

To a solution of diisopropylamine (20.26 mL, 142 mmol, 3.90 equiv.) in THF (80 mL) was added at-78°C n-buthyllithium (1.6 M in hexane, 86 mL, 138 mmol, 3.77 equiv.) and the mixture stirred for 15 min at -78°C. Then at-78°C a solution of methyl acetate (10.95 mL, 137 mmol, 3.77 equiv.) in THF (40 mL) was added dropwise and the mixture stirred for another 20 min at that temperature. A solution of β-lactone **2** (7.155 g, 36.5 mmol, 1.00 equiv.) in THF (50 mL) was added and the mixture kept stirring at-78°C for 15 h. After consumption of the starting material the reaction was quenched with a saturated aqueous solution of NH₄Cl (70 mL). The layers were separated and the aqueous layer extracted with EtOAc (3 x 100 mL). The combined organic layers were washed with brine, dried over MgSO₄, filtered and concentrated *in vacuo.* Purification by flash column chromatography (SiO₂, hexane/EtOAc, 7:1 to 1:1 gradient) gave the β-ketoester 3 (7.57 g, 28.0 mmol, 77%) as a 14:1 mixture together with its tautomer (peaks of the minor tautomer are marked with an asterisk) as a pale yellow oil.

TLC: *R_{f}* = 0.34 (hexane/EtOAc, 4:1, vanilline). **¹H NMR** (400 MHz, CDCl₃): δ = 12.15* (s, 1 H), 5.46 - 5.26 (m, 2H), 5.06* (s, 1 H), 4.09 (m, 1 H), 4.02 - 3.93* (m, 2H), 3.74 (s, 3H), 3.70* (s, 2H) 3.64* (s, 1 H), 3.49 (s, 2H), 2.75* (d, *J* = 3.3 Hz, 3H), 2.72 - 2.64 (m, 2H), 2.63* (d, *J* = 8.5 Hz, 3H), 2.20 - 2.08 (m, 2H), 2.03 (q, *J* = *6.7* Hz, 2H), 1.66 - 1.51 (m, 1 H), 1.50 - 1.39 (m, 1 H), 1.38 - 1.22 (m, 7H), 0.89 (t, *J* = 6.9 Hz, **3H). ¹³C NMR** (100 MHz, CDCl₃): δ = 203.58, 175.75*, 167.46, 131.20, 128.65, 90.99*, 69.15*, 67.27, 52.60, 51.39*, 49.83, 49.80, 43.01*, 36.99*, 36.45, 31.66, 29.51, 27.35, 23.40, 22.72, 14.21. **IR** (thin film): 3447, 2925, 2863, 1750, 1709, 1653, 1630, 1438, 1400, 1321, 1152, 1072, 1005, 858, 771 cm⁻¹. **HRMS** (ESI): exact mass calculated for C₁₅H₂₆O₄ [(M + Na)⁺]: 293.1723; found: 293.1727. **[a]_{D}^{26°C} = -** 24.51 (c = 0.94, CHCl₃).

### Example 3: (R,Z)-Methyl 2-diazo-5-hdyroxy-3-oxotetradec-8-enoate

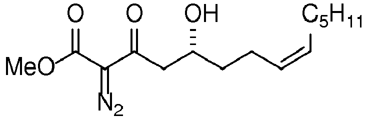

To a solution of β-ketoester **3** (6.98 g, 25.8 mmol, 1.00 equiv.) in MeCN (200 mL) at 0 °C was added sequentially 4-acetamidobenzenesulfonyl azide (8.31 g, 33.6 mmol, 1.30 equiv.) and Et₃N (7.26 mL, 51.6 mmol, 2.00 equiv.). The mixture was allowed to warm to room temperature and stirred for 5.5 h. The reaction mixture became turbid during that time and was filtered through a plug of celite before it was concentrated *in vacuo.* The residue was taken up in CH₂Cl₂ (80 mL) and filtered again through a plug of celite. The solvent was removed *in vacuo* and the residue purified by flash column chromatography (SiO₂, hexane/EtOAc, 5:1) to yield the diazo compound (7.39 g, 24.9 mmol, 97%) as yellow oil.

**TLC:** *R_{f}* = 0.53 (hexane/EtOAc, 4:1, vanilline, UV). **¹H NMR** (400 MHz, CDCl₃): δ = 5.41 - 5.27 (m, 2H), 4.11 - 4.01 (m, 1 H), 3.81 (s, 3H), 3.08 - 2.97 (m, 2H), 2.90 (dd, *J* = 17.2, 9.1 Hz, 1 H), 2.22 - 2.06 (m, 2H), 2.00 (q, *J* = 6.6 Hz, 2H), 1.64 - 1.41 (m, 2H), 1.36 - 1.18 (m, 6H), 0.85 (t, *J* = 6.9 Hz, 3H). **¹³C NMR** (100 MHz, CDCl₃): 5 = 192.87, 161.72, 130.88, 128.80, 67.71, 52.37, 46.90, 46.86, 36.65, 31.58, 29.46, 27.25, 23.38, 22.64, 14.12. **IR** (thin film): 3474, 2922, 2845, 2131, 1718, 1641, 1437, 1313, 1199, 1132, 1080, 1027, 741. **HRMS** (ESI): exact mass calculated for C₁₅H₂₄N₂O₄[(M + Na)⁺]: 319.1634; found: 319.1628. [α]_{D}^{23°C} = - 38.1 (c = 1.31, CHCl₃).

### Example 4: (R,Z)-Methyl 2-diazo-3-oxo-5-((triethylsilyl)oxy)tetradec-8-enoate (4)

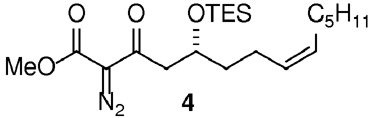

To a solution of the diazo compound 3.39 g, 11.4 mmol, 1.00 equiv.) in DMF (22 mL) at 0°C was added imidazole (1.55 g, 22.8 mmol, 2.00 equiv.) and triethylchlorosilane (2.90 mL, 17.3 mmol, 1.51 equiv.). The mixture was allowed to warm to room temperature and stirred for 3 h. Then the reaction mixture was diluted with Et₂O (150 mL) and washed with water (400 mL). The organic layer was dried over MgSO₄, filtered and concentrated *in vacuo*. Purification by flash column chromatography (SiO₂, hexane/EtOAc, 8:1) gave the TES-protected diazo compound **4** (4.59 g, 11.18 mmol, 98%) as yellow oil.

**TLC:** *R_{f}* = 0.65 (hexane/EtOAc, 5:1, vanilline, **UV). ¹H NMR** (400 MHz, CDCl₃): δ = 5.40 - 5.30 (m, 2H), 4.31 -4.20 (m, 1 H), 3.83 (s, 3H), 3.15 (dd, *J* = 15.5, 7.2 Hz, 1 H), 2.92 (dd, *J* = 15.6, 5.3 Hz, 1 H), 2.18 - 1.97 (m, 4H), 1.61 - 1.48 (m, 2H), 1.39 - 1.19 (m, 6H), 0.99 - 0.81 (m, 12H), 0.58 (q, *J* = 7.9 Hz, 6H). **¹³C NMR** (100 MHz, CDCl₃): δ = 191.03, 161.78, 130.56, 129.13, 68.88, 52.34, 47.42, 38.15, 31.69, 29.53, 27.36, 23.17, 22.73, 14.22, 6.99, **5.10. IR** (thin film): 2955, 2876, 2133, 1725, 1657, 1436, 1374, 1314, 1196, 1086, 1002, 743 cm⁻¹. **HRMS** (ESI): exact mass calculated for C₂₁H₃₈N₂O₄Si [(M + Na)⁺]: 433.2493; found: 433.2498. [α]_{D}^{24°C} = - 15.8 (c = 0.95, CHCl₃).

### Example 5: (1R,2R,3S)-Methyl 2-((Z)-oct-2-en-1-yl)-5-oxo-3-((triethylsilyl)oxy)-cyclopentanecarboxlayte (5)

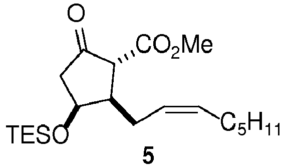

To a solution of Rh₂(S-PTAD)₄ (170 mg, 0.109 mmol, 0.01 equiv.) in CH₂Cl₂ (200 mL) at reflux was added a solution of TES-protected diazo compound **4** (4.48 g, 10.9 mmol, 1.00 equiv.) in CH₂Cl₂ (30 mL). The mixture was stirred at reflux for 20 min and then allowed to cool to room temperature. The reaction mixture was concentrated *in vacuo* and purified by flash column chromatography (SiO₂, hexane/Et₂O, 9:1) to yield cyclic β-ketoester **5** (2.96 g, 7.75 mmol, 71%) in a 9:1 mixture of diastereomers as yellow oil. The mixture was usually used as such, since the diastereomers were more easily separated after the next step. For characterization however, the diastereomers were separated by column chromatography. Around 8% of the product was present in the enol form. Assignable signals of the enol tautomer are marked with an asterisk *.

TLC: *R_{f}* = 0.29 (hexane/Et₂O, 4:1, Vanillin, UV). **¹H NMR** (400 MHz, CDCl₃): δ = 12.24* (s, 1 H), 5.58 - 5.25 (m, 2H), 4.45 (t, *J* = 3.8 Hz, 1H), 3.71 (s, 3H), 3.64* (s, 3H), 3.15 (d, *J* = 11.6 Hz, 1H), 2.81 - 2.67* (m, 2H), 2.65 - 2.53 (m, 1 H), 2.50 (dd, *J* = 18.0, 4.2 Hz, 1 H), 2.44 - 2.33 (m, 2H), 2.31 - 2.18 (m, 1H), 2.01 (q, *J* = 7.0 Hz, 2H), 1.39 - 1.21 (m, 6H), 0.93 (t, *J* = 7.9 Hz, 9H), 0.87 (t, *J* = 7.0 Hz, 3H), 0.59 (q, *J* = 7.8 Hz, **6H). ¹³C NMR** (100 MHz, CDCl₃): δ = 210.20, 170.18, 132.28, 126.17, 69.90, 57.71, 52.46, 49.46, 47.93, 31.62, 29.31, 27.41, 26.99, 22.66, 14.16, 6.89, 4.97. **IR** (thin film): 2952, 2867, 1758, 1730, 1459, 1431, 1355, 1336, 1150, 1126, 1074, 1050, 1003, 827, 732 cm⁻¹. **HRMS** (ESI): exact mass calculated for C₂₁H₃₈O₄Si [(M + Na)⁺]: 405.2432; found: 405.2432. [a]_{D}^{25°C} = - 5.7 (c = 0.82, CHCl₃).

### Example 6: (3R.4S)-3-((Z)-Oct-2-en-1-yl)-4-((triethylsilyl)oxy)cyclopentanone

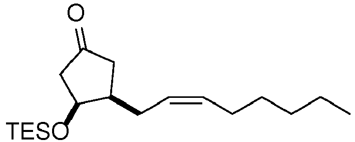

A solution of cyclic β-ketoester 5 (2.69 g, 7.05 mmol, 1.00 equiv.) and sodium chloride (12.36 g, 211 mmol, 30 equiv.) in degassed DMSO (100 mL) was immersed into an oil bath pre-heated to 140°C and stirred for 30 min. Then the mixture was allowed to cool to room temperature and diluted with water (900 mL). It was extracted with EtOAc (3 x 250 mL) and the combined organic layers were dried over MgSO₄, filtered and concentrated *in vacuo.* Purification by flash column chromatography (SiO₂, hexane/EtOAc, 98:2) allowed the separation of the cis-substituted cyclopentanone (1.49 g, 4.59 mmol, 65%) from the *trans*-product (0.17 g, 0.524 mmol, 7%).

**TLC:** *R_{f}* = 0.71 (hexane/EtOAc, 5:1, vanilline). **¹H NMR** (400 MHz, CDCl₃): δ = 5.51 - 5.28 (m, 2H), 4.49 - 4.35 (m, 1 H), 2.43 - 1.92 (m, 9H), 1.43 - 1.17 (m, 6H), 0.95 (t, *J* = 7.9 Hz, 9H), 0.89 (t, *J* = 6.9 Hz, 3H), 0.60 (q, *J* = 8.0 Hz, 6H). ¹³C **NMR** (100 MHz, CDCl₃): δ = 217.76, 131.63, 127.30, 71.66, 49.53, 43.69, 41.17, 31.67, 29.46, 27.51, 27.49, 22.70, 14.21, 6.96, 5.04. **IR** (thin film): 2955, 2916, 2869, 1747, 1455, 1403, 1236, 1150, 1116, 1078, 1040, 1006, 739 cm⁻¹. **HRMS** (EI): exact mass calculated for C₁₉H₃₆O₂Si [M⁺]: 324.2485; found: 324.2483. [aj_{D}^{25°C} = - 54.5 (c = 1.02, CHCl₃).

### Example 7: (R,Z)-4-(Oct-2-en-1-yl)cyclopent-2-enone (6)

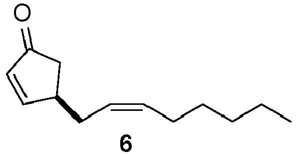

To a solution of the cis-substituted cyclopentanone (1.21 g, 3.73 mmol, 1.00 equiv.) in CH₂Cl₂ (35 mL) at 0°C was added 1,8-Diazabicyclo[5.4.0]undec-7-ene (5.60 mL, 37.2 mmol, 9.97 equiv.) and the mixture stirred at 0°C for 15 h. The mixture was diluted with a saturated aqueous solution of NH₄Cl (30 mL) and allowed to warm to room temperature. The layers were separated and the aqueous was extracted with CH₂Cl₂ (3 x 50 mL). The combined organic layers were washed with brine, dried over Na₂SO₄, filtered and concentrated *in vacuo.* In order to remove the residuing silanol the mixture was azeotroped with toluene (5 x 15 mL). Purification by flash column chromatography (SiO₂, hexane/EtOAc, 7:1) gave cyclopentenone **6** (667 mg, 3.47 mmol, 93%) as a pale yellow oil.

**TLC:** *R_{f}* = 0.52 (hexane/EtOAc, 5:1, vanilline, UV). **¹H NMR** (400 MHz, CDCl₃): δ = 7.62 (dd, *J* = 5.6, 2.5 Hz, 1 H), 6.16 (dd, *J* = 5.6, 2.0 Hz, 1 H), 5.60 - 5.43 (m, 1 H), 5.40 - 5.26 (m, 1 H), 3.04 - 2.96 (m, 1 H), 2.51 (dd, *J* = 18.9, 6.4 Hz, 1 H), 2.39 - 2.11 (m, 2H), 2.07 - 1.91 (m, 3H), 1.41 - 1.19 (m, 6H), 0.87 (t, *J* = 6.9 Hz, 3H). **¹³C NMR** (100 MHz, CDCl₃): δ = 209.98, 168.13, 134.19, 133.01, 125.50, 41.57, 40.65, 32.08, 31.62, 29.36, 27.44, 22.66, 14.16. **IR** (thin film): 2922, 2845, 2356, 1717, 1582, 1457, 1404, 1347, 1179, 780 cm⁻¹. **HRMS** (EI): exact mass calculated for C₁₃H₂₀O[M⁺]: 192.1509; found: 192.1508. [a]_{D}^{25°C} = + 1.3 (c = 0.82, CHCl₃).

### Example 8: Methyl 4-((2R,3R)-3-((E)-((S)-2-((Z)-oct-2-en-1-yl-5-oxocyclopent-3-en-1-ylidene)-methyl)oxiran-2-yl)butanoate

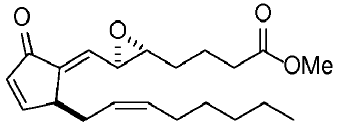

To a solution of LiHMDS (503 mg, 3.01 mmol, 1.20 equiv.) in THF (10 mL) at-78°C was added a solution of cyclopentenone **6** (482 mg, 2.50 mmol, 1.00 equiv.) in THF (2 x 3 mL) and the mixture stirred for 30 min. Then a solution of epoxy aldehyde 7 (863 mg, 5.01 mmol, 2.00 equiv.) in THF (2 x 4 mL) was added and the mixture stirred at -78 °C for 1.5 h. The reaction was quenched at -78°C with a saturated, aqueous solution of NH₄Cl (25 mL) and the layers were separated. The aqueous layer was extracted with ether (3 x 40 mL) and the combined organic layers were washed with brine, dried over MgSO₄, filtered and concentrated *in vacuo.* The crude product was purified by flash column chromatography (SiO₂, hexane/EtOAc, 2:1) to yield a mixture of the aldol product (2 isomers) and the co-spotting aldehyde 7. Without further purification the mixture was dissolved in CH₂Cl₂ (20 mL) and treated at -78°C with Et₃N (2.1 mL, 15.05 mmol, 6.00 equiv.) and methanesulfonyl chloride (0.59 mL, 7.52 mmol, 3.00 equiv.). After 1 h at -78°C the reaction mixture was directly filtered through a pad of silica into a 50 mL-round-bottom flask. The silica pad was carefully washed with dry CH₂Cl₂ (2 x 10 mL) and to the resulting solution of the mesylate was added neutral aluminum oxide (2.56 g, 25.08 mmol, 10.00 equiv.). The mixture was vigorously stirred at room temperature for 18 h and then filtered through a plug of celite. Then the mixture was concentrated *in vacuo* and purified by flash column chromatography (SiO₂, hexane/EtOAc, 4:1 to 3:1 gradient) to give the dienone (445 mg, 1.29 mmol, 64%) as a yellow oil.

TLC: *R_{f}=* 0.58 (hexane/EtOAc, 2:1, vanilline, UV). **¹H NMR** (300 MHz, CDCl₃): δ = 7.54 (ddd, *J* = 6.0, 2.6, 0.9 Hz, 1 H), 6.35 (dd, *J* = 6.0, 1.8 Hz, 1 H), 6.19 (dt, *J* = 8.2, 1.1 Hz, 1 H), 5.59 - 5.45 (m, 1 H), 5.41 - 5.26 (m, 1 H), 3.65 (s, 3H), 3.39 (dd, *J* = 8.2, 2.1 Hz, 1 H), 2.99 (ddd, *J* = 6.4, 4.0, 2.1 Hz, 1 H), 2.64 - 2.47 (m, 1 H), 2.44 - 2.25 (m, 3H), 2.05 - 1.92 (m, 2H), 1.90 - 1.71 (m, 3H), 1.68 - 1.49 (m, 2H), 1.42 - 1.17 (m, 6H), 0.88 (t, *J* = 6.9 Hz, 3H). **¹³C NMR** (100 MHz, CDCl₃): δ = 195.87, 173.59, 162.05, 141.24, 134.59, 133.52, 131.06, 124.53, 60.03, 55.05, 51.78, 43.45, 33.59, 32.02, 31.67, 31.37, 29.34, 27.48, 22.69, 21.49, 14.20. **IR** (thin film): 2924, 2847, 2361, 1736, 1703, 1656, 1579, 1436, 1202, 1169, 1102, 1016, 863 cm⁻¹. **HRMS** (ESI): exact mass calculated for C₂₁H₃₀O₄[(M + Na)⁺]: 369.2036; found: 369.2030. [α]_{D}^{23°C} = + 178.6 (c = 0.22, CHCl₃).

### Example 9: 4-((2R,3R)-3-((E)-((S)-2-((Z)-Oct-2-en-1-yl)-5-oxocyclopent-3-en-1-ylidene)-methyl)oxiran-2-yl)butanoic acid, EC(8)

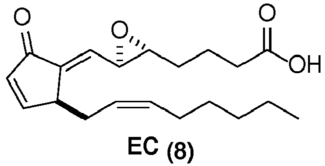

To a solution of the dienone methyl ester (80 mg, 0.231 mmol, 1.00 equiv.) in THF (0.92 mL) and aqueous phosphate buffer pH 7 (3.70 mL) at room temperature was added Novozyme® (30 mg, lipase on acrylic resin from *Candida Antarctica*) and the mixture stirred for 1 h. The mixture was filtered through a plug of cotton and the filtrate treated with EtOAc (10 mL). The layers were separated and the pH of the aqueous layer was carefully adjusted to pH 5 using 0.1 M HCl. Then the aqueous layer was extracted with EtOAc (3 x 10 mL) and the combined organic layers were dried over MgSO₄, filtered and concentrated *in vacuo.* Purification by flash column chromatography (SiO₂, hexane/ EtOAc/MeOH, 4:1:1) gave **EC** (**8**) (54 mg, 0.162 mmol, 70%) as a pale yellow oil.

**TLC:** *R_{f}* = 0.40 (hexane/EtOAc/MeOH, 4:1:1, vanilline, **UV). ¹H NMR** (400 MHz, CDCl₃): δ = 7.54 (ddd, *J* = 6.0, 2.6, 0.9 Hz, 1 H), 6.35 (dd, *J* = 6.0, 1.8 Hz, 1 H), 6.19 (dt, *J* = 8.1, 1.0 Hz, 1 H), 5.58 - 5.46 (m, 1 H), 5.41 - 5.24 (m, 1 H), 3.69 - 3.64 (m, 1 H), 3.40 (dd, *J* = 8.2, 2.0 Hz, 1 H), 3.01 - 2.98 (m, 1 H), 2.58 - 2.51 (m, 1 H), 2.45 (t, *J* = 6.0 Hz, 2H), 2.32 (dt, *J* = 15.2, 8.3 Hz, 1 H), 1.98 (q, *J* = 7.2 Hz, 2H), 1.89 - 1.74 (m, 3H), 1.72 - 1.53 (m, 1 H), 1.43 - 1.12 (m, 6H), 0.88 (t, *J* = 7.0 Hz, 3H). **¹³C NMR** (100 MHz, CDCl₃): δ = 195.90, 177.41, 162.11, 141.29, 134.58, 133.54, 130.98, 124.53, 60.00, 55.06, 43.46, 33.29, 32.02, 31.67, 31.29, 29.34, 27.48, 22.68, 21.28, 14.19. **IR** (thin film): 2918, 2850, 2365, 1703, 1657, 1574, 1555, 1456, 1205 cm⁻¹. **HRMS** (ESI): exact mass calculated for C₂₀H₂₈O₄[(M - H)⁻]:331.1915; found: 331.1910. [α]_{D}^{24°C} = + 127.9 (c = 0.66, CHCl₃).

### Example 10: (S)-6-((R,E)-1-Hydroxy-2-((S)-2-((Z)-oct-2-en-1-yl)-5-oxocyclopent-3-en-1-liydene)-ethyl)tetrahydro-2H-pyran-2-one, cycloEC (9)

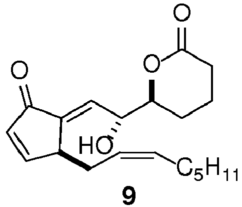

To a solution of **EC** (**8**) (11.9 mg, 0.036 mmol, 1.00 equiv.) in CHCl₃ (2 mL) at room temperature was added silica gel (100 mg) and the mixture stirred for 3 h. Then the mixture was filtered through a pad of celite and the filter cake carefully washed with EtOAc (3x). The filtrate was concentrated *in vacuo* and purified by flash column chromatography (SiO₂, hexane/acetone, 4:1 to 3:1 gradient) to yield the cycloEC **9** (7.2 mg, 0.021 mmol, 65%) as a colorless oil.

Other acids tested for cyclisation proved to be far less suitable:

| *Reagent* | *Condition* | *Result* |
|---|---|---|
| CSA | CH₂Cl₂, 0°C | side product, only small amount of product |
| CSA | CHCl₃, 0°C | side product, only small amount of product |
| pTsOH | THF, rt | no reaction |
| pTsOH | CH₂Cl₂, rt | decomposition |
| pTsOH | CHCl₃, 0°C | decomposition |
| PPTS | CH₂Cl₂, rt | no reaction |
| BF₃ OEt₂ | CH₂Cl₂, 0°C | side product, only small amount of product |
| Sc(OTf)₃ | CH₂Cl₂, 0°C | side product, only small amount of product |
| Sc(OTf)₃ | EtOH/H₂O, rt | no reaction |

TLC: *R_{f}* = 0.27 (hexane/acetone, 7:3, vanilline, UV). **¹H NMR** (400 MHz, CDCl₃): δ=7.57 (ddd, *J* = 6.0, 2.6, 0.8 Hz, 1 H), 6.40 - 6.35 (m, 2H), 5.53 - 5.47 (m, 1 H), 5.31 - 5.25 (m, 1 H), 4.82 - 4.78 (m, 1 H), 4.45 - 4.41 (m, 1 H), 3.78 - 3.74 (m, 1 H), 2.68 - 2.60 (m, 2H), 2.52 - 2.44 (m, 2H), 2.33 - 2.25 (m, 1 H), 2.03 - 1.95 (m, 3H), 1.90 - 1.78 (m, 3H), 1.36 - 1.20 (m, 6H), 0.88 (t, *J* = 6.9 Hz, 3H). **¹³C NMR** (100 MHz, CDCl₃): 5 = 196.70, 171.08, 163.35, 140.99, 134.27, 133.50, 128.39, 124.49, 82.57, 70.76, 43.64, 31.66, 31.28, 29.88, 29.33, 27.52, 22.69, 21.52, 18.52, 14.19. **IR** (thin film): 2920, 1725, 1700, 1651, 1344, 1237, 1203, 1179, 1047, 931, 804 cm⁻¹. **HRMS** (ESI): exact mass calculated for C₂₀H₂₈O₄ [(M + NH₄)⁺]: 350.2326; found: 350.2334. [α]_{D}^{22°C} = + 178.5 (c = 0.31, CHCl₃).

### Biological experiments:

### Example 11: Co-culture experiment shown in Figure 1

Spleens from wildtype C57BL/6 mice were digested in 1 mg/ml Collagenase IV (BIOCONCEPT-Worthington, LS004189) for 45 minutes at 120 rpm in 12-well plates at 37°C. Spleens were then processed through 70 µm cell strainer and washed with PBS before MACS sorting with CD11c+ specific beads (Miltenyi Biotec, 130-052-001). Naive CD62L^{hi}, Va2⁺ CD4 T cells from SMARTA2 transgenic mice were obtained by MACS sorting of spleen with CD4+ beads (Miltenyi Biotec, 130-049-201). Bead labeled cells were then separated on LS Separation Columns (Miltenyi Biotec, 130-042-401) according to manufacturer's guidelines. Dendritic cells were left to equilibrate for 60 min at 5% CO₂, 37°C then treated with 500nM cyclo-EC (**9**) for 60 minutes, washed with medium and co-cultured together with CD4+ T cells in the presence of 10 nM LCMV derived peptide gp61 for 4 days. Cells were then re-stimulated with PMA (Fluka-Sigma, P-8139), lonomycin (Cayman Chemical, 10004974) and treated with Brefeldin A (Fluka-Sigma, B7651) for 4 h. Polarization of T cells was assessed by intracellular FACS staining for IL-4 (Biolegend, 504104, Clone 11B11) and IFNγ (Biolegend, 505810, XMG1.2)

### Example 12: Expression of Nrf2 target genes and of pro-inflammatory chemokine genes in bone marrow derived dendritic cells pretreated with cyclo-EC (9) (Figure 2)

Bone marrow cells from tibia and femur were isolated *ex vivo* and differentiated *in vitro* into bone marrow derived dendritic cells (BMDC) by cultivation in the presence of 2 ng/ml carrier free recombinant mouse GM-CSF (BioLegend, 576308) in RPMI 1640 medium (GE Healthcare, E15-039) supplemented with L-glutamine (Lonza-Cambrex BioWhittaker, BE17-605E), HEPES Buffer (Lonza-Cambrex BioWhittaker, BE17-737E), peniciline/ streptomycine (Gibco Life Technologies 15140-122) and fetal bovine serum (Gibco Life Sciences 10270-106). Fresh RPMI-1640 GM-CSF supplemented medium was added at days 3 and 5 of culture. Cells were harvested at day 7, counted and seeded into 12-well flat bottom plates at 10⁶ cells/well. After 1 h of equilibration at 37°C and 5% CO₂, cyclo-EC (9) or control solvent was added to the wells for 75 minutes. Cells were washed thereafter with PBS and stimulated for 3 h with 5 µg/ml R837 (3700, Tocris). Cells were then lysed by addition of 1 ml of TRIzol Reagent (Ambion Life Technologies, 15596018) per well and transferred in 2 ml Eppendorf tubes. Cells were incubated for 5 minutes at room temperature. mRNA was purified according to manufacturer's instructions: 100 µl bromo-chloropropane (Sigma-Aldrich, BP-151) was added to each sample and after 10 min of incubation at room temperature, lysate was centrifuged for 15 min at 12'000 g and subsequently precipitated with isopropanol and washed with EtOH. The pellet was resuspended in water and reverse transcribed into cDNA. Potentially contaminating genomic DNA was first digested by RNAse free DNAse (Life Technologies, AM2222) and 2 µg mRNA/reaction was reverse transcribed by GoScript™ Reverse Transcriptase (Promega, A5004) in presence of RNAse inhibitor (Bioconcept, M0314L). Quantitative PCR was performed using KAPA SYBR FAST Bio-Rad iCycler Kit (LabgeneScientific SA, KK4608) and expression was normalized to house keeping gene G6PDH.

### Example 13: IL-6 and IL-12 expression in bone marrow derived dendritic cells pretreated with compounds 8 (EC), 9 (cEC) and 10 to 18 (Figures 3 to 5)

Bone marrow cells from tibia and femur were isolated *ex vivo* and differentiated *in vitro* into bone marrow derived dendritic cells (BMDC) by cultivation in the presence of 2 ng/ml carrier free recombinant mouse GM-CSF (BioLegend, 576308) in RPMI 1640 medium (GE Healthcare, E15-039) supplemented with L-glutamine (Lonza-Cambrex BioWhittaker, BE17-605E), HEPES Buffer (Lonza-Cambrex BioWhittaker, BE17-737E), peniciline/ streptomycine (Gibco Life Technologies 15140-122) and fetal bovine serum (Gibco Life Sciences 10270-106). Fresh GM-CSF supplemented RPMI-1640 medium was added at days 3 and 5. Cells were then harvested, counted and seeded into 96-well flat bottom plates at 10⁵ cells/well. Meanwhile lipids **8-18** indicated in Figures 3 and 4 were prepared by serial 1:2 dilution in separate 96-well plates. After 1 h of equilibration lipids were added for 60 minutes to the wells containing BMDCs. Cells were then washed twice with PBS to get rid of residual lipids in supernatants and cells were stimulated overnight with 5 µg/ml R837 (3700, Tocris). Supernatants were harvested after 18 h. Cytokine concentrations in supernatant were quantified by sandwich ELISA using the following antibody pairs: IL-6 (504502 and 504602) from BioLegend and IL-12 (14-7125-85 and 13-7123-85) from eBioscience. For detection of biotinylated secondary antibody, Streptavidin-AP (7100-04) from SouthernBiotech and colorimetric substrate pNPP (N2765-100TAB) from Sigma-Aldrich were used. Optical density was measured using a Spectra MAX 190 device by Bucher biotec.

In the experiments shown in Figure 5, EC **8** and cEC **9** were prepared by serial 2:3 dilution in separated 96-well plates in RPMI-1640. After 1 h of equilibration the compounds were added to the wells containing BMDCs for 60 minutes.

## Claims

1. A compound of formula (I) or (II) 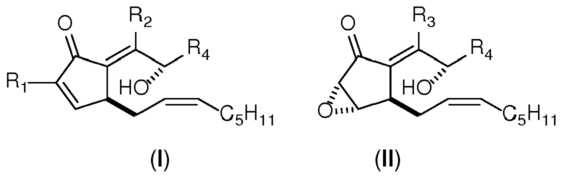 wherein
R₁, R₂ and R₃ are, independently of each other, hydrogen, fluoro, chloro, nitro or trifluoromethyl;
R₄ is of partial formula 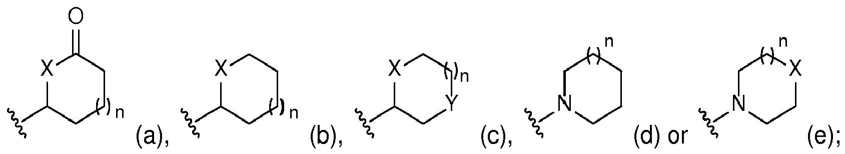
X and Y, are independently of each other, O or NR₅;
R₅ is hydrogen or C₁-C₄-alkyl; and
n is 0 or 1.

2. The compound of formula (I) or (II) wherein R₄ is of partial formula (a).

3. The compound according to claim 1 or 2 of formula (III) or (IV) 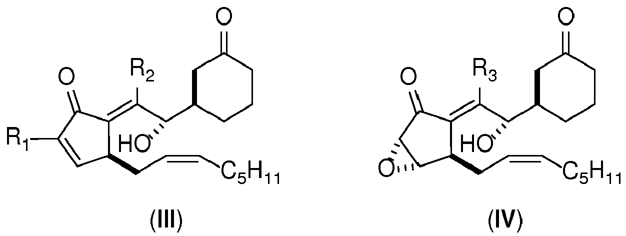 wherein R₁, R₂ and R₃ are, independently of each other, hydrogen, fluoro, chloro, nitro or trifluoromethyl.

4. The compound according to any one of claims 1 to 3 of formula (**I**) or (**III**) wherein R₁ and R₂ are, independently of each other, hydrogen, fluoro, chloro, nitro or trifluoromethyl.

5. The compound according to any one of claims 1 to 4 of formula (**I**) or (**III**) wherein R₁ is hydrogen, fluoro, chloro, nitro or trifluoromethyl, and R₂ is hydrogen.

6. The compound according to any one of claims 1 to 5 of formula (**I**) or (**III**) wherein R₁ is hydrogen, fluoro or chloro, and R₂ is hydrogen.

7. The compound according to any one of claims 1 to 6 of formula (**I**) or (**III**) wherein R₁ and R₂ are hydrogen.

8. The compound according to claim 1 or 2 of formula (**II**) or (**IV**), wherein R₃ is hydrogen, fluoro, chloro, nitro or trifluoromethyl.

9. The compound according to claim 1, 2 or 8 of formula (**II**) or (**IV**), wherein R₃ is hydrogen, chloro or fluoro.

10. The compound according to claim 1, 2, 8 or 9 of formula (**II**) or (**IV**), wherein R₃ is hydrogen.

11. A pharmaceutical composition comprising a compound according to any one of claims 1 to 10.

12. A method of synthesis of compounds of formula (**III**) and (**IV**), 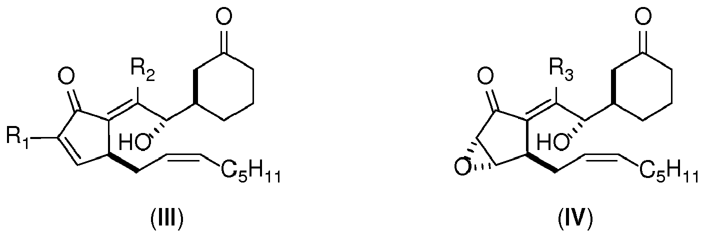 wherein R₁, R₂ and R₃ are, independently of each other, hydrogen, fluoro, chloro, nitro or trifluoromethyl.
**characterized in that** a compound of formula (**V**) and (**VI**), respectively, 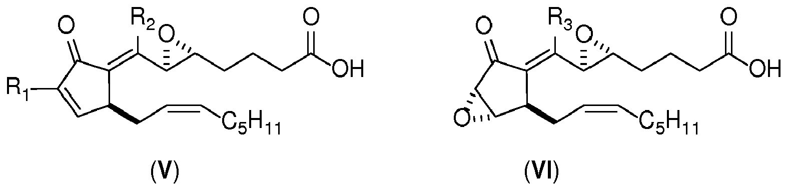 wherein R₁, R₂ and R₃ have the meaning indicated for formula (**III**) and (**IV**), is treated with SiO₂,

13. The compound according to any one of claims 1 to 10 for use in the treatment of a bacterial infection and inflammation.

14. The compound according to any one of claims 1 to 10 for use in the treatment of psoriasis, atopic dermatitis, inflammatory bowel disease, arthritis, sepsis, asthma, sarcoidosis, acute respiratory distress syndrome, diabetes mellitus, multiple sclerosis, cancer, atherosclerosis, vasculitis, glomerulonephritis, or steatosis.

15. A method of treatment of bacterial infection or inflammation, which comprises administering a compound according to any one of claims 1 to 10 in a quantity effective against said disease, to a warm-blooded animal requiring such treatment.
